# EUROPÄISCHE PATENTSCHRIFT

(11) **EP 1 013 638 B1**
(45) Veröffentlichungstag und Bekanntmachung des Hinweises auf die Patenterteilung: **13.11.2002**
(21) Anmeldenummer: 99124029.2
(22) Anmeldetag: 09.12.1999
(51) Int. Cl.: C07C 263/10

(54) **Verfahren zur Herstellung von Duroldiisocyanat**
Method of producing duroldiisocyanate
Procédé pour la préparation d'isocyanate de durol

(30) Priorität: 22.12.1998 DE 19859297
(43) Veröffentlichungstag der Anmeldung: 28.06.2000
(73) Patentinhaber: BAYER AG, 51368 Leverkusen (DE)
(72) Erfinder: Heitkämper, Peter, Dr., 41542 Dormagen (DE); Jost, Klaus, Dr., 41539 Dormagen (DE); Penninger, Stefan, Dr., 50259 Pulheim (DE)

(56) Entgegenhaltungen:
- DE-A- 3 317 649
- US-A- 3 089 862
- US-A- 3 153 099

## Beschreibung

Die Erfindung betrifft ein verbessertes Verfahren zur Herstellung von Duroldiisocyanat (= 2,3,5,6-Tetramethyl-1,4-diisocyanatobenzol) durch Umsetzung von Durol (= 1,2,4,5-Tetramethylbenzol) mit Salpetersäure in Schwefelsäure, katalytische Hydrierung des so hergestellten Dinitrodurols (= 2,3,5,6-Tetramethyl-1,4-dinitrobenzol), und Phosgenierung des so hergestellten Duroldiamins (= 2,3,5,6-Tetramethyl-1,4-diaminobenzol).

Die Herstellung von Duroldiisocyanat ist bekannt. In der Britischen Patentschrift 779 806 ist ein Verfahren beschrieben, bei dem Duroldiamin in Chlorbenzol aufgelöst, dann mit Chlorwasserstoff-Gas zu Duroldiamindihydrochlorid umgesetzt und dieses als Suspension in Chlorbenzol bei erhöhter Temperatur mit Phosgen weiter umgesetzt wird.

Duroldiamin ist seit langem bekannt und wird üblicherweise aus Dinitrodurol durch Reduktion hergestellt. Die Hydrierung von Dinitrodurol zu Duroldiamin in Ethanol unter Zusatz von Raney-Nickel als Katalysator ist beispielsweise im Journal of the American Chemical Society , Band 70, Seite 2227, sowie Band 72, Seite 132 beschrieben.

Dinitrodurol ist ebenfalls seit langem bekannt und wird üblicherweise durch Nitrierung von Durol hergestellt. In der US-Patentschrift 3 153 099 ist ein verbessertes Verfahren zur Herstellung von Dinitrodurol beschrieben, bei dem eine Suspension von Durol in konzentrierter wäßriger Schwefelsäure in Abwesenheit von organischen Lösungsmitteln bei Temperaturen von 5 bis 10°C mit einem Salpetersäure-Schwefelsäure-Wasser-Gemisch umgesetzt, dann die resultierende Suspension in ein Eis-Wasser-Gemisch eingerührt und das Dinitrodurol abfiltriert, mit Wasser gewaschen und bei 60 bis 70°C getrocknet wird.

Wie diese Patentschrift lehrt, ist die an sich naheliegende Verfahrensweise, Durol in einem organischen Lösungsmittel zu lösen und diese Lösung einer Nitrierung zu unterwerfen, mit erheblichen Nachteilen, vor allem mit geringen Ausbeuten an Dinitrodurol und hohen Anteilen an Nebenprodukten verbunden. Dieser Umstand wird auch durch das Beispiel 1 (Vergleichsbeispiel) der vorliegenden Patentschrift bestätigt.

Zwar stellt die Nitrierung von Durol in schwefelsaurer Suspension eine Verbesserung gegenüber der Nitrierung einer Lösung in einem organischen Lösungsmittel dar. Jedoch ist das in der US-Patentschrift 3 153 099 beschriebene Verfahren mit großen Nachteilen behaftet. So hat der als bevorzugt genannte Bereich der Reaktionstemperatur von 5 bis 10°C unwirtschaftlich lange Reaktionszeiten zur Folge. Das bestätigt auch das Beispiel 2 (Vergleichsbeispiel) der vorliegenden Patentschrift.

Ein weiterer Nachteil des beschriebenen Verfahrens besteht darin, daß das fertige Reaktionsgemisch in ein Eis-Wasser-Gemisch eingerührt wird. Diese Verwendung von Eis zur Verdünnung von konzentrierter Schwefelsäure mit Wasser zur Bewältigung der dabei auftretenden Mischungswärme ist wohl für Arbeiten im Labormaßstab eine übliche und praktikable Methode, ist aber für großtechnische Verfahren ungeeignet. Sie erfordert nämlich die Bereitstellung und Handhabung großer Mengen an Eis, verbunden mit einem erheblichen technischen Aufwand für die Herstellung und den Transport des Eises sowie für die Verarbeitung des Eises in großvolumigen Behältern, gegebenenfalls mit vergleichsweise starken Rührmotoren. Weiterhin hat dieser Verfahrensschritt zur Folge, daß die gesamte zur Umsetzung eingesetzte Schwefelsäure verdünnt wird und deshalb als wertlose Abfallsäure entsorgt oder aufwendig wieder aufkonzentriert werden muß.

Ein weiterer Nachteil des in der US-Patentschrift 3 153 099 beschriebenen Verfahrens besteht darin, daß das abfiltrierte rohe Dinitrodurol nur durch Waschen mit Wasser gereinigt wird, so daß die in den Dinitrodurol-Partikeln eingeschlossenen Verunreinigungen, z.B. Schwefelsäure, nicht abgetrennt werden. Andererseits ist insbesondere im Hinblick auf die auch in dieser US-Patentschrift genannte Verwendung von Dinitrodurol (Spalte 3, Zeilen 47 bis 50), nämlich die Herstellung von Duroldiamin durch Reduktion, eine Mindestqualität des Dinitrodurols notwendig. Das bedeutet im allgemeinen, daß das so hergestellte Dinitrodurol in einem weiteren Verfahrensschritt gereinigt werden muß, beispielsweise durch Umkristallisation.

Als technisches Verfahren zur Reduktion von Dinitrodurol zu Duroldiamin ist nur die katalytische Hydrierung realistisch. Andere bekannte Verfahren, wie die Umsetzung von Dinitrodurol mit Ammoniumsulfid, mit Zink oder mit Zinn(II)chlorid eignen sich wohl für Umsetzungen im Labormaßstab, kommen aber für eine großtechnische Herstellung von Duroldiamin aus wirtschaftlichen und ökologischen Gründen nicht in Frage.

Die beschriebenen Verfahren der katalytischen Hydrierung in einem protischen Lösungsmittel wie Ethanol sind im Hinblick auf die Umsetzung von Duroldiamin mit Phosgen mit dem Nachteil behaftet, daß das Lösungsmittel aus dem Reaktionsgemisch praktisch vollständig entfernt werden muß, weil anderenfalls bei der Phosgenierung unerwünschte Reaktionsprodukte des Lösungsmittels mit Phosgen entstehen. Das bedeutet aber, daß in einem aufwendigen Verfahrensschritt die Lösung von Duroldiamin vollständig eingedampft und anschließend das zurückbleibende feste Duroldiamin in einem aprotischen Lösungsmittel wieder aufgelöst werden muß;

Das in der Britischen Patentschrift 779 806 beschriebene Verfahren zur Herstellung von Duroldiisocyanat durch Umsetzung von Duroldiamindihydrochlorid mit Phosgen ist ebenfalls mit großen Nachteilen verbunden. So ist die Herstellung von Duroldiamindihydrochlorid mit Chlorwasserstoff-Gas kosten- und zeitaufwendig. Ebenso ist die Umsetzung des Dihydrochlorids mit Phosgen in Suspension langwierig und hat außerdem zur Folge, daß das Prozeßabgas mit vergleichsweise großen Mengen an Chlorwasserstoff belastet ist.

Ziel der vorliegenden Erfindung ist es daher, ein technisches Verfahren zur Herstellung von Duroldiisocyanat zur Verfügung zu stellen, das die genannten Nachteile vermeidet und bei dem die einzelnen Reaktionsstufen und Verfahrensschritte aufeinander abgestimmt sind.

Gegenstand der vorliegenden Erfindung ist deshalb ein verbessertes Verfahren zur Herstellung von Duroldiisocyanat durch Umsetzung von Durol mit Salpetersäure in Schwefelsäure, katalytische Hydrierung des so hergestellten Dinitrodurols und Phosgenierung des so hergestellten Duroldiamins, das dadurch gekennzeichnet ist, daß man a) nach Beendigung der Umsetzung von Durol mit Salpetersäure in Schwefelsäure bei 10-15°C die im wesentlichen aus Schwefelsäure bestehende flüssige Phase des Reaktionsgemisches durch Vermischen mit Wasser verdünnt, b) die so hergestellte Suspension mit einem mit Wasser praktisch nicht mischbaren und gegenüber Wasserstoff inerten aprotischen organischen Lösungsmittel intensiv vermischt, dadurch das enthaltene Dinitrodurol in dem Lösungsmittel auflöst und so ein Gemisch mit zwei. flüssigen Phasen enthält, c) aus diesem Gemisch die Lösung von Dinitrodurol in dem Lösungsmittel durch Phasentrennung separiert und gegebenenfalls anschließend durch Extraktion mit Wasser reinigt, d) die so erhaltene Dinitrodurol-Lösung, gegebenenfalls nach Aufkonzentrieren durch destillative Abtrennung von Lösungsmittel, unter Zusatz eines festen, unlöslichen Katalysators einer Hydrierung unterwirft, e) aus dem Reaktionsgemisch dieser Hydrierung das Wasser und den Katalysator abtrennt und f) die verbleibende Lösung des so hergestellten Duroldiamins einer Phosgenierung unterwirft.

Es muß als äußerst überraschend angesehen werden, daß das erfindungsgemäße Verfahren insgesamt so hohe Ausbeuten an Duroldiisocyanat ergibt, obwohl bei der zugrunde liegenden Dreistufenreaktion die Zwischenprodukte Dinitrodurol und Duroldiamin nicht isoliert und gesondert gereinigt werden. Insbesondere ist der Umstand überraschend, daß Dinitrodurol aus dem Reaktionsgemisch der Umsetzung von Durol mit Salpetersäure durch einfaches Lösen in einem aprotischen Lösungsmittel in solcher Reinheit abgetrennt werden kann, daß die anschließende katalytische Hydrierung erfolgreich durchgeführt werden kann.

Bei dem erfindungsgemäßen Verfahren wird die Umsetzung von Durol mit Salpetersäure in Schwefelsäure weitgehend entsprechend den Angaben der US-Patentschrift 3 153 099 durchgeführt, jedoch nicht die Aufarbeitung des hergestellten Dinitrodurols.

Durol wird in feinteiliger Form eingesetzt, um eine möglichst vollständige Umsetzung in Suspension zu erzielen.

Zur Herstellung der Suspension wird bei dem erfindungsgemäßen Verfahren Durol mit der 5- bis 20-fachen, vorzugsweise 7- bis 15-fachen Gewichtsmenge an konzentrierter Schwefelsäure, die einen Wasser-Gehalt von 2 bis 40 Gew.-%, vorzugsweise 4 bis 20 Gew.-% besitzt, vermischt.

Zur Umsetzung mit Durol kann reine Salpetersäure verwendet werden. Es können aber auch Gemische von Salpetersäure mit Schwefelsäure und/oder Wasser zum Einsatz gelangen, wobei dann der Gehalt dieser Gemische an Schwefelsäure 0 bis 75 Gew.-% und der Gehalt an Wasser 0 bis 40 Gew.-% beträgt.

Bei der Umsetzung wird die Salpetersäure in Mengen von 2,0 bis 2,2 mol, bevorzugt 2,0 bis 2,1 mol, bezogen auf 1 mol Durol, eingesetzt.

Die Umsetzung von Durol mit Salpetersäure wird bei dem erfindungsgemäßen Verfahren bei Temperaturen von 10 bis 15°C, bevorzugt bei 11 bis 13°C, durchgeführt.

Bei einer besonderen Ausführungsform des erfindungsgemäßen Verfahrens wird aus dem fertigen Reaktionsgemisch der Umsetzung von Durol mit Salpetersäure ein wesentlicher Anteil der flüssigen Phase mittels Filtrieren oder Zentrifugieren des Dinitrodurols abgetrennt. Der Anteil der so abgetrennten Absäure beträgt im allgemeinen 50 bis 95 Gew.-%, vorzugsweise 60 bis 90 Gew.-% der gesamten flüssigen Phase und ist im wesentlichen durch die Wirksamkeit des Filtrier- oder Zentrifugierprozesses begrenzt.

Bei einer weiteren besonderen Ausführungsform des erfindungsgemäßen Verfahrens wird die auf diese Weise erhaltene Absäure wieder für eine Folgeumsetzung von Durol mit Salpetersäure verwendet. Dazu wird im allgemeinen die Absäure vor der Umsetzung mit zusätzlichen Mengen an konzentrierter wäßriger oder gegebenenfalls an reiner Schwefelsäure vermischt. Die Konzentration und die Menge der zugegebenen Schwefelsäure hängen von der Menge und Zusammensetzung der abgetrennten Absäure ab. Im allgemeinen wird man aber bestrebt sein, für die einzelnen Umsetzungen von Durol mit Salpetersäure annähernd die gleichen Mengenverhältnisse von Durol, Schwefelsäure und Wasser einzusetzen.

Nach dem erfindungsgemäßen Verfahren wird das fertige Reaktionsgemisch der Umsetzung von Durol mit Salpetersäure mit Wasser vermischt. Wenn nicht nach der besonderen Ausführungsform verfahren wird, nämlich vor dem Vermischen mit Wasser das hergestellte rohe Dinitrodurol durch Filtrieren oder Zentrifugieren abzutrennen, ist es im allgemeinen zweckmäßig, das Wasser vorzulegen und das Reaktionsgemisch in das Wasser einzurühren. Es kann aber auch von Vorteil sein, in einem Mischaggregat das fertige Reaktionsgemisch in einem kontinuierlichen Strom mit einem Wasser-Strom zu vermischen und dabei das Gemisch zu kühlen.

Wenn entsprechend der besonderen Ausführungsform rohes Dinitrodurol vor dem Vermischen mit Wasser durch Filtrieren oder Zentrifugieren abgetrennt wird, ist es zweckmäßig, mit dem Wasser den Dinitrodurol-Filterkuchen aufzuschlämmen. Wenn es gewünscht wird, kann durch den Wasserfluß gleichzeitig auch die entstehende Suspension in einen anderen Behälter gefördert werden.

Die zum Vermischen eingesetzte Menge an Wasser hängt im wesentlichen davon ab, mit wieviel Schwefelsäure das rohe Dinitrodurol vermischt ist. Üblicherweise wird dabei so viel Wasser verwendet, daß die resultierende Flüssigkeit weniger als 40 Gew.-%, vorzugsweise weniger als 30 Gew.-%, Schwefelsäure enthält. Im allgemeinen ist es zweckmäßig, mindestens so viel Wasser zu verwenden, daß die Temperatur des resultierenden Gemisches nicht mehr als 100°C beträgt. Um nicht eine unwirtschaftlich große Wassermenge zu verwenden, kann es von Vorteil sein, die Vermischung mit Wasser unter wirksamer äußerer Kühlung durchzuführen. Die Kühlung ist aber im allgemeinen nicht erforderlich, wenn das Reaktionsgemisch vor dem Vermischen mit Wasser durch Filtrieren oder Zentrifugieren entsprechend der oben ausgeführten Verfahrensweise von einem wesentlichen Anteil der flüssigen Phase befreit worden ist.

Die durch Vermischen mit Wasser hergestellte Suspension von rohem Dinitrodurol wird bei dem erfindungsgemäßen Verfahren mit einem mit Wasser praktisch nicht mischbaren aprotischen organischen Lösungsmittel intensiv vermischt. Als Lösungsmittel sind dabei solche Solventien geeignet, die sich gegenüber wäßriger Schwefelsäure und unter den Bedingungen der katalytischen Hydrierung und unter den Bedingungen der Phosgenierung sowie gegenüber Duroldiisocyanat inert verhalten. Beispielhaft seien als geeignete Lösungsmittel genannt: Isooctan, Waschbenzin, Dekahydronaphthalin, Toluol, m-Xylol, 1,2,3,4-Tetrahydronaphthalin, Chlorbenzol, o-Dichlorbenzol, 2-Chlortoluol und 1-Chlornaphthalin. Auch Gemische von geeigneten Lösungsmitteln können bei dem erfindungsgemäßen Verfahren verwendet werden. Vorzugsweise wird jedoch Toluol oder Chlorbenzol als Lösungsmittel verwendet.

Bei dem erfindungsgemäßen Verfahren kann das intensive Vermischen der Dinitrodurol-Suspension mit dem organischen Lösungsmittel bei tiefen Temperaturen erfolgen. Zweckmäßig wird das Vermischen jedoch bei erhöhter Temperatur durchgeführt, um den Lösevorgang zu beschleunigen. Dabei liegt die Temperatur jedoch unterhalb des Siedepunktes des betreffenden Lösungsmittels. Sie beträgt im allgemeinen 30 bis 95°C. Es kann von Vorteil sein, die Temperatur der wäßrigen Dinitrodurol-Suspension durch Wahl einer entsprechend geringen Wassermenge beim Vermischen auf etwa 90 bis 95°C einzustellen, um auf diese Weise beim Lösen in dem Lösungsmittel die angestrebte erhöhte Temperatur zu erzielen. Selbstverständlich ist es auch möglich, die Temperatur durch Einsatz von erhitztem Lösungsmittel und/oder durch äußere Beheizung beim Lösen zu erhöhen.

Die Menge des zum Auflösen des Dinitrodurols eingesetzten Lösungsmittels hängt vom Lösungsvermögen des Solvens und von der Temperatur des Gemisches ab und kann leicht durch entsprechende Vorversuche bestimmt werden. Zweckmäßig wird nicht wesentlich mehr Lösungsmittel verwendet, als zur Lösung des Dinitrodurols erforderlich ist. Eine hohe Verdünnung mit Lösungsmittel ist im allgemeinen nachteilig, weil dann zur Herstellung und Verarbeitung der Lösung Behälter mit großem Volumen erforderlich sind. Üblich sind Mengen an Lösungsmittel im Bereich von 3 bis 100 kg, bevorzugt 4 bis 20 kg, bezogen auf 1 kg hergestelltes Dinitrodurol.

Die Trennung der Dinitrodurol-Lösung von der wäßrigen Phase erfolgt bei dem erfindungsgemäßen Verfahren durch Phasentrennung. Dabei ist die zur ausreichenden Trennung erforderliche Zeit stark abhängig von der Zusammensetzung der Phasen und insbesondere von der Art des verwendeten Lösungsmittels. Zweckmäßig werden für eine möglichst rasche Phasentrennung die Konzentrationen der Phasen so gewählt, daß die Dichten der Phasen deutlich unterschiedlich sind.

Nach der Abtrennung der organischen Phase kann es von Vorteil sein, sie durch intensives Vermischen mit Wasser und erneute Phasentrennung zu reinigen. Das ist besonders dann vorteilhaft, wenn die organische Phase wegen schleppender Phasentrennung noch einen signifikanten Anteil an emulgierter wäßriger Säure enthält.

Für die Hydrierung des Dinitrodurols können stark verdünnte Lösungen eingesetzt werden. Im allgemeinen ist es aber zweckmäßig, möglichst konzentrierte Lösungen zu hydrieren, um hohe Raum-Zeit-Ausbeuten zu erzielen. Daher kann es vorteilhaft sein, aus den Dinitrodurol-Lösungen vor dem Hydrieren einen Teil des Lösungsmittels destillativ abzutrennen. Lösungen von Dinitrodurol mit einer Konzentration von Dinitrodurol von 8 bis 40 Gew.-% sind daher vorteilhaft.

Bei dem erfindungsgemäßen Verfahren werden die Lösungen von Dinitrodurol in Gegenwart von festen, im Lösungsmittel praktisch nicht löslichen Katalysatoren hydriert. Dabei kommen Katalysatoren zum Einsatz, wie sie in der Technik zur katalytischen Hydrierung von Nitroaromaten üblich sind. Beispielhaft seien hier genannt: Raney-Nickel, Raney-Nickel-Eisen und Raney-Kobalt. Edelmetall-Katalysatoren wie Palladium oder Platin können zwar grundsätzlich auch verwendet werden, kommen aber im allgemeinen aus wirtschaftlichen Gründen und wegen der Gefahr der Kernhydrierung weniger in Betracht.

Die Menge des zur Hydrierung eingesetzten Katalysators ist abhängig von der Art des Katalysators, von der Art des Lösungsmittels sowie von der Konzentration und der Reinheit des gelösten Dinitrodurols und variiert in einem weiten Bereich. Das optimale Mengenverhältnis kann leicht durch orientierende Vorversuche ermittelt werden. Üblich sind dabei Mengen an Katalysator im Bereich von 0,1 bis 10 kg, bezogen auf 100 kg zu hydrierendes Dinitrodurol.

Die Reaktionstemperatur bei der katalytischen Hydrierung der Dinitrodurol-Lösung innerhalb des erfindungsgemäßen Verfahrens ist ebenfalls abhängig von der Art des eingesetzten Katalysators und der Art des verwendeten Lösungsmittels und variiert in einem Bereich von 50 bis 250°C.

Bei dem erfindungsgemäßen Verfahren wird die katalytische Hydrierung der Dinitrodurol-Lösung bei einem Wasserstoffdruck von 1,1 bis 200 bar, vorzugsweise 3 bis 100 bar, durchgeführt.

Das fertige Reaktionsgemisch der katalytischen Hydrierung stellt ein Mehrphasengemisch dar, das neben der Lösung von Duroldiamin in dem organischen Lösungsmittel noch den Katalysator als feste Phase sowie im allgemeinen als zweite flüssige Phase Wasser aus der Hydrierreaktion enthält. Vor dem Einsatz der Duroldiamin-Lösung in die Phosgenierung werden bei dem erfindungsgemäßen Verfahren das Wasser und der Katalysator zweckmäßigerweise vollständig aus dem Reaktionsgemisch entfernt.

Bei einer besonderen Ausführungsform wird vor der Abtrennung des Katalysators das Reaktionsgemisch durch teilweise Destillation vom Wasser befreit. Wenn die Hydrierung bei einer Temperatur oberhalb des Siedepunktes des Lösungsmittels durchgeführt wird, kann es vorteilhaft sein, das fertige Reaktionsgemisch durch Entspannungsverdampfung abzukühlen. Dadurch werden einerseits die Entspannung des verwendeten Druckreaktors und die gewünschte Abkühlung des Gemisches, andererseits auch eine Teildestillation zur Abtrennung von Wasser ohne zusätzlichen Energie-Aufwand erreicht. Falls erforderlich, wird dann nach dem Druckausgleich noch so viel Lösungsmittel zusammen mit Wasser abdestilliert, bis das Reaktionsgemisch praktisch wasserfrei ist. Das kann direkt nach der Entspannungsverdampfung im Druckreaktor erfolgen, so daß kein zusätzlicher Destillationsbehälter erforderlich ist.

Die Abtrennung des Katalysators aus dem Reaktionsgemisch der Hydrierung oder aus der wasserfreien Duroldiamin-Lösung erfolgt bei dem erfindungsgemäßen Verfahren nach den in der Technik üblichen Methoden, beispielsweise durch Filtrieren oder Zentrifugieren.

Die Umsetzung der wasser- und katalysatorfreien Lösung von rohem Duroldiamin mit Phosgen in üblichen Mengen (ca. 2 bis 8 mol, vorzugsweise 3 bis 6 mol Phosgen, bezogen auf 1 mol Duroldiamin) zum Duroldiisocyanat kann bei dem erfindungsgemäßen Verfahren nach Art der üblichen Kalt-Heiß-Phosgenierung durchgeführt werden. Das bedeutet, daß in dem Phosgenierreaktor eine Lösung von Phosgen in dem z.B. zuvor eingesetzten organischen Lösungsmittel bei niedriger Temperatur (ca. 0 bis 30°C) vorgelegt wird, die Duroldiamin-Lösung, gegebenenfalls unter Kühlung, eingemischt wird, anschließend die resultierende Suspension zum Rückfluß erhitzt wird und schließlich die Umsetzung, gegebenenfalls unter Zugabe von weiterem Phosgen am Rückfluß vervollständigt wird.

Eine besondere Ausführungsform des erfindungsgemäßen Verfahrens besteht darin, daß die gegebenenfalls heiße Duroldiamin-Lösung in einem mechanisch betriebenen Mischaggregat kontinuierlich mit einer Lösung von Phosgen in dem Lösungsmittel intensiv vermischt wird, die resultierende Suspension in einen nachgeschalteten Reaktor gefördert wird und dort die Umsetzung zu Ende geführt wird. Dabei sind als Mischaggregate die in der Technik üblichen Geräte geeignet, beispielsweise Mischpumpen oder Stachelmischer. Durch die Verwendung eines Mischaggregats wird aufgrund der Scherkräfte eine gleichmäßige Vermischung und damit eine Minimierung der Nebenprodukte erreicht. Außerdem muß dann das Reaktionsgemisch nicht gekühlt werden, sondern kann in dem nachgeschalteten Reaktor direkt zum Rückfluß erhitzt werden.

Nach Beendigung der Phosgenierung wird das Reaktionsgemisch üblicherweise durch destillative Abtrennung des noch enthaltenen Phosgens zusammen mit einem Teil des Lösungsmittels aufgearbeitet. Das hergestellte Duroldiisocyanat kann dann, wie in der Britischen Patentschrift 779806 beschrieben, durch Auskristallisieren gewonnen und durch Umkristallisieren gereinigt werden. Zweckmäßig wird das Diisocyanat jedoch destillativ gewonnen und durch fraktionierte Destillation gereinigt. Duroldiisocyanat kann unzersetzt bei Temperaturen unterhalb von 200°C destilliert werden. Dazu sind Vakua erforderlich, wie sie ohne Schwierigkeiten großtechnisch erzeugt werden können, z.B. Drücke von 1 bis 22 mbar.

Die destillative Aufarbeitung des Reaktionsgemisches hat gegenüber der Aufarbeitung mittels Kristallisation den Vorteil, daß sie deutlich weniger aufwendig ist und einen höheren Reinheitsgrad des Duroldiisocyanats ergibt. Außerdem wird das Lösungsmittel in reiner Form gewonnen und kann so wieder zur Lösung des rohen Dinitrodurols eingesetzt werden. Schließlich kann bei der fraktionierten Destillation auch ein gegebenenfalls im Rohprodukt noch enthaltener kleiner Anteil an nicht umgesetztem Durol in reiner Form wiedergewonnen und zur Nitrierung miteingesetzt werden.

Das erfindungsgemäße Verfahren kann, insgesamt oder in einzelnen Teilschritten, kontinuierlich oder diskontinuierlich durchgeführt werden.

Als wesentliche Vorteile des erfindungsgemäßen Verfahrens gegenüber dem Stand der Technik seien genannt:
- Die Zwischenprodukte Dinitrodurol und Duroldiamin werden nicht aufwendig als Festprodukte, sondern in gelöster Form verarbeitet.
- Die Zwischenprodukte Dinitrodurol und Duroldiamin werden nicht aufwendig isoliert und gereinigt, sondern direkt nach der Herstellung weiter verarbeitet.
- Anstelle der aufwendigen Verwendung von Eis wird Wasser zum Verdünnen der Schwefelsäure eingesetzt.
- Durch Verwendung von nur einem organischen Lösungsmittel für den gesamten Herstellungsprozeß wird die Anzahl der erforderlichen technischen Einrichtungen wie Lagerbehälter und Destillationskolonnen minimiert.
- Durch die Abtrennung und Wiederverwendung von Absäure bei der Umsetzung von Durol mit Salpetersäure ist das Verfahren deutlich weniger umweltbelastend.

Das nach dem erfindungsgemäßen Verfahren hergestellte Duroldiisocyanat stellt ein wertvolles Ausgangsprodukt zur Herstellung von Polyurethan-Kunststoffen dar.

### Beispiele

Die folgenden Beispiele sollen das erfindungsgemäße Verfahren näher erläutern:

### Beispiel 1: Vergleichsbeispiel

In einem 21-Rundkolben, der mit einem Tropftrichter versehen war, wurde eine Lösung von 100 g Durol in 600 ml n-Hexan vorgelegt. In die Lösung wurden innerhalb von einer Stunde unter Rühren bei 20 bis 25°C 100 g Salpetersäure (100 %-ig) eingetropft. Dabei wurde die Reaktionswärme durch eine Eisbad-Kühlung kompensiert. Anschließend wurde das Reaktionsgemisch noch zwei Stunden bei 25°C gerührt und dann in einem Scheidetrichter mit 500 ml Wasser ausgeschüttelt. Nach Abtrennung der Wasserphase wurde die organische Phase destillativ von n-Hexan befreit, wobei eine klare gelbe Flüssigkeit zurückblieb. Nach gaschromatografischer und infrarotspektroskopischer Analyse enthielt das Reaktionsprodukt neben wenig Durol mehrere nicht näher identifizierte Verbindungen. Dinitrodurol konnte in dem Reaktionsprodukt-Gemisch nicht nachgewiesen werden.

### Beispiel 2: Vergleichsbeispiel

In einem Emaille-Kessel, der mit einem Emaille-Rührer und einer externen Sole-Kühlung ausgestattet war, wurden 110 kg einer 95,8 prozentigen Schwefelsäure vorgelegt, auf 5°C abgekühlt und unter Rühren mit 10,0 kg gemahlenem Durol versetzt. Das Gemisch wurde 15 Minuten gerührt, wobei eine homogene Suspension entstand. In die Suspension wurden unter schwacher Kühlung bei 5 bis 7°C innerhalb von 15 Minuten 2,1 kg (entsprechend 6,4 % der stöchiometrisch erforderlichen Menge) einer Mischsäure (28,4 Gew.-% Salpetersäure; 57,6 Gew.-% Schwefelsäure; 14,0 Gew.-% Wasser) eingerührt. Dabei wurde nur eine geringe Reaktionswärme beobachtet. Deshalb wurde die Mischsäure-Zugabe abgebrochen. Aus dem Reaktionsgemisch wurde eine Probe entnommen, in Eis eingerührt und dann analysiert. Dabei zeigte sich, daß der Salpetersäure-Umsatz nur 36 % betragen hatte.

### Beispiel 3: Herstellung von Duroldiisocyanat

In einem Emaille-Kessel, der mit einem Emaille-Rührer und einer externen Sole-Kühlung ausgestattet war, wurden 136 kg konzentrierte Schwefelsäure (89,8 Gew.-% Schwefelsäure; 10,2 Gew.-% Wasser) vorgelegt, auf 10°C abgekühlt und unter Rühren mit 11,4 kg gemahlenem Durol versetzt. Das Gemisch wurde 15 Minuten gerührt, wobei eine homogene Suspension entstand. In diese Suspension wurden innerhalb von 2,8 Stunden unter guter Sole-Kühlung und kräftigem Rühren 32,5 kg Nitriersäure (33 Gew.-% Salpetersäure; 67 Gew.-% Schwefelsäure) so zudosiert, daß die Reaktionstemperatur 11 bis 13°C betrug. Anschließend wurde das breiige Gemisch noch eine Stunde bei 11 bis 13°C gerührt. In einem weiteren Kessel mit Rührer und externer Kühlung wurden 400 1 Wasser von 15°C vorgelegt. Das Reaktionsgemisch wurde dann unter Rühren und Kühlen so in das Wasser eindosiert, daß die resultierende Temperatur nicht über 95°C anstieg. Die so erhaltene gut rührbare Suspension wurde mit 100 l Chlorbenzol versetzt und 30 Minuten lang intensiv verrührt. Nach der anschließenden Phasentrennung (30 Minuten) wurde die untere, wäßrige Phase abgetrennt. Aus der zurückbleibenden organischen Phase wurden 40 1 Chlorbenzol abdestilliert. Die so aufkonzenrierte Lösung wurde mit 400 g Raney-Nickel versetzt und in einem Autoklaven bei 180°C und 40 bis 50 bar hydriert. Nach 4,5 Stunden war die Wasserstoff-Aufnahme beendet. Der Autoklav wurde durch Entspannungsverdampfung auf 130°C abgekühlt. Aus dem Reaktionsgemisch wurden dann noch so lange Chlorbenzol und Wasser abdestilliert, bis das Gemisch frei von Wasser war (insgesamt 22 l Chlorbenzol). Dann wurde das Reaktionsgemisch unter Zugabe von heißem wasserfreiem Chlorbenzol (insgesamt 14 1) durch Filtrieren über eine Drucknutsche bei 110 bis 120°C von Raney-Nickel befreit. Das klare 110°C heiße Filtrat wurde über einen Stachelmischer mit 110 kg einer kalten 25 gew.-%igen Lösung von Phosgen in Chlorbenzol vermischt. Das resultierende Reaktionsgemisch wurde in einen Kessel gegeben, unter schwacher Phosgen-Einleitung zum Rückfluß erhitzt und noch eine Stunde am Rückfluß phosgeniert, wobei eine klare Lösung entstand. Das Reaktionsgemisch wurde destillativ von überschüssigem Phosgen und von Chlorbenzol befreit. Das zurückbleibende Rohprodukt wurde im Vakuum bei 18 mbar flashdestilliert. Dabei wurden 13,5 kg gelbliches Destillat erhalten, das nach gaschromatografischer Analyse neben 2,7 % Durol 97,0 % Duroldiisocyanat enthielt. Das Produkt wurde durch fraktionierte Vakuumdestillation gereinigt und ergab ein farbloses Destillat vom Siedepunkt 182-184°C bei 18 mbar, das zu Kristallen vom Schmelzpunkt 113-114°C erstarrte.

### Beispiel 4: Herstellung von Duroldiisocyanat

Ebenso wie in Beispiel 3 wurden 11,4 kg Durol in 136 kg konzentrierter Schwefelsäure mit 32,5 kg Nitriersäure umgesetzt. Nach der Zugabe der Nitriersäure wurde das Reaktionsgemisch noch eine Stunde bei 11 bis 13°C gerührt und dann über eine Drucknutsche mittels Stickstoffdruck filtriert. Dabei wurden 125 kg Absäure (88,3 Gew.-% Schwefelsäure; 10,3 Gew.-% Wasser) als klare dunkel gefärbte Flüssigkeit erhalten. Der Filterkuchen wurde mit 120 1 Wasser von 15°C aufgeschlämmt und in einen Kessel gespült. Die so erhaltene gut rührbare Suspension von 70°C wurde mit 100 l Chlorbenzol versetzt und 30 Minuten lang intensiv verrührt. Die weiteren Verfahrensschritte wurden wie in Beispiel 3 durchgeführt. Es wurden 13,7 kg flashdestilliertes Duroldiisocyanat vom Gehalt 96,8 Gew.-% erhalten.

### Beispiel 5: Herstellung von Duroldiisocyanat

Duroldiisocyanat wurde ebenso wie in Beispiel 4 hergestellt, wobei jedoch zur Nitrierung von Durol (11,4 kg) die bei der Filtration von rohem Dinitrodurol erhaltene Absäure aus Beispiel 4 (125 kg; 88,3 Gew.-% Schwefelsäure) verwendet wurde, die vor der Umsetzung mit zusätzlichen 13 kg konzentrierter Schwefelsäure (7,0 Gew.-% Wasser) vermischt wurde. Nach Beendigung der Nitrierung wurden bei der Filtration von Dinitrodurol 128 kg Absäure (87,3 Gew.-% Schwefelsäure; 10,2 Gew.-% Wasser) erhalten.

Die Flashdestillation des rohen Duroldiisocyanats ergab 13,5 kg gelbliches Produkt mit einem Gehalt von 2,6 % Durol und 96,5 % Duroldiisocyanat.

### Beispiel 6: Herstellung von Duroldiisocyanat

Ebenso wie in Beispiel 3 wurden 11,4 kg Durol in 136 kg konzentrierter Schwefelsäure mit 32,5 kg Nitriersäure umgesetzt. Das fertige Reaktionsgemisch wurde in 400 1 Waser eingerührt. Die erhaltene Suspension wurde bei 70°C mit 120 l Toluol 45 Minuten lang intensiv vermischt. Nach der anschließenden Phasentrennung wurde die untere, wäßrige Phase abgetrennt. Aus der zurückbleibenden organischen Phase wurden 60 l Toluol abdestilliert. Die so aufkonzenrierte Lösung wurde mit 400 g Raney-Nickel versetzt und in einem Autoklaven bei 170°C und 41 bis 54 bar hydriert. Nach 4 Stunden war die Wasserstoff-Aufnahme beendet. Der Autoklav wurde durch Entspannungsverdampfung auf 110°C abgekühlt. Aus dem Reaktionsgemisch wurden dann noch so lange Toluol und Wasser abdestilliert, bis das Gemisch frei von Wasser war. Dann wurde das Reaktionsgemisch durch Filtrieren über eine Drucknutsche bei 100 bis 110°C von Raney-Nickel befreit. Das klare 100°C heiße Filtrat wurde über einen Stachelmischer mit 110 kg einer kalten 25 gew.-%igen Lösung von Phosgen in Toluol vermischt. Das resultierende Reaktionsgemisch wurde in einen Kessel gegeben, unter schwacher Phosgen-Einleitung zum Rückfluß erhitzt und noch zwei Stunden am Rückfluß phosgeniert, wobei eine klare Lösung entstand. Das Reaktionsgemisch wurde destillativ von überschüssigem Phosgen und von Toluol befreit. Das zurückbleibende Rohprodukt wurde im Vakuum bei 18 mbar flashdestilliert. Dabei wurden 13,6 kg Duroldiisocyanat mit einer Reinheit von 96,9 % erhalten.

Wie die Beispiele 3 bis 6 zeigen, können bei der Dreistufenreaktion zur Herstellung von Duroldiisocyanat nach dem erfindungsgemäßen Verfahren in technisch einfacher Weise hohe Ausbeuten an Duroldiisocyanat erzielt werden.

## Patentansprüche

1. Verfahren zur Herstellung von Duroldiisocyanat durch Umsetzung von Durol mit Salpetersäure in Schwefelsäure, in Abwesenheit eines orgänischen Lösungsmittels, katalytische Hydrierung des so hergestellten Dinitrodurols und Phosgenierung des so hergestellten Duroldiamins, **dadurch gekennzeichnet, daß** man a) nach Beendigung der Umsetzung von Durol mit Salpetersäure in Schwefelsäure in einem Temperaturbereich von 10 bis 15°C die im wesentlichen aus Schwefelsäure bestehende flüssige Phase des Reaktionsgemisches durch Vermischen mit Wasser verdünnt, b) die so hergestellte Suspension mit einem mit Wasser nicht mischbaren und gegenüber Wasserstoff inerten aprotischen organischen Lösungsmittel intensiv vermischt, dadurch das enthaltene Dinitrodurol in dem Lösungsmittel auflöst und so ein Gemisch mit zwei flüssigen Phasen enthält, c) aus diesem Gemisch die Lösung von Dinitrodurol in dem organischen Lösungsmittel durch Phasentrennung separiert und gegebenenfalls anschließend durch Extraktion mit Wasser reinigt, d) die so erhaltene Dinitrodurol-Lösung, gegebenenfalls nach Aufkonzentrieren durch destillative Abtrennung von Lösungsmittel, unter Zusatz eines festen, unlöslichen Katalysators einer Hydrierung unterwirft, e) aus dem Reaktionsgemisch dieser Hydrierung das Wasser und den Katalysator abtrennt und f) die verbleibende Lösung des so hergestellten Duroldiamins einer Phosgenierung unterwirft.

2. Verfahren gemäß Anspruch 1, **dadurch gekennzeichnet, daß** man aus dem fertigen Reaktionsgemisch der Umsetzung von Durol mit Salpetersäure vor dem Vermischen mit Wasser einen Teil der flüssigen Phase durch Filtrieren oder Zentrifugieren des Dinitrodurols abtrennt.

3. Verfahren gemäß Anspruch 3, **dadurch gekennzeichnet, daß** man die beim Filtrieren oder Zentrifugieren des rohen Dinitrodurols aus dem Reaktionsgemisch abgetrennte Absäure wieder für eine Folgeumsetzung von Durol mit Salpetersäure einsetzt.

4. Verfahren gemäß Anspruch 1, **dadurch gekennzeichnet, daß** man als aprotisches organisches Lösungsmittel Toluol einsetzt.

5. Verfahren gemäß Anspruch 1, **dadurch gekennzeichnet, daß** man als aprotisches organisches Lösungsmittel Chlorbenzol einsetzt.

6. Verfahren gemäß Anspruch 1, **dadurch gekennzeichnet, daß** man nach Beendigung der katalytischen Hydrierung des gelösten Dinitrodurols zunächst das Reaktionsgemisch durch Destillation vollständig von dem enthaltenden Wasser befreit, dann aus dem verbleibenden Reaktionsgemisch den Katalysator abtrennt und schließlich die verbleibende Lösung von Duroldiamin, gegenenfalls nach Verdünnung mit dem gleichen Lösungsmittel, einer Phosgenierung unterwirft.

7. Verfahren gemäß Anspruch 1, **dadurch gekennzeichnet, daß** man zur Phosgenierung die Lösung des durch die Hydrierung gewonnenen Duroldiamins nach der Abtrennung des Wassers und des Katalysators, gegebenenfalls nach Verdünnung mit dem gleichen Lösungsmittel, kontinuierlich in einem mechanisch betriebenen Mischaggregat intensiv mit einer Lösung von Phosgen in einem organischen Lösungsmittel vermischt, das resultierende Reaktionsgemisch in einen nachgeschalteten Reaktor gibt und dort die Umsetzung zu Duroldiisocyanat durch Temperaturerhöhung und gegebenenfalls durch Zugabe von weiterem Phosgen zu Ende führt.

## Claims

1. Method for preparing durol diisocyanate by reacting durol with nitric acid in sulfuric acid, in the absence of an organic solvent, catalytic hydrogenation of the thus prepared dinitrodurol, and phosgenation of the resultant durol diamine, **characterised in that** a) after completion of the reaction of durol with nitric acid in sulfuric acid in a temperature range from 10 to 15°C the liquid phase of the reaction mixture, which consists substantially of sulfuric acid, is diluted by mixing with water, b) the resultant suspension is intensively mixed with an aprotic organic solvent that is immiscible with water and is inert with respect to hydrogen, the contained dinitrodurol thereby dissolving in the solvent with the formation of a mixture consisting of two liquid phases, c) the solution of dinitrodurol in the organic solvent is separated from this mixture by phase separation and optionally is then purified by extraction with water, d) the dinitrodurol solution thus obtained is subjected to a hydrogenation under the addition of a solid, insoluble catalyst, optionally after concentration by distillative separation of solvents, e) the water and the catalyst are separated from the reaction mixture of this hydrogenation, and f) the remaining solution of the resultant durol diamine is subjected to a phosgenation.

2. Method according to claim 1, **characterised in that** part of the liquid phase is separated from the end reaction mixture of the reaction of durol with nitric acid before mixing with water, by filtration or centrifugation of the dinitrodurol.

3. Method according to claim 2, **characterised in that** the spent acid separated from the reaction mixture in the filtration or centrifugation of the crude dinitrodurol is reused for a subsequent reaction of durol with nitric acid.

4. Method according to claim 1, **characterised in that** toluene is used as aprotic organic solvent.

5. Method according to claim 1, **characterised in that** chlorobenzene is used as aprotic organic solvent.

6. Method according to claim 1, **characterised in that** after completion of the catalytic hydrogenation of the dissolved dinitrodurol the reaction mixture is first of . all completely freed from the contained water by distillation, the catalyst is then separated from the remaining reaction mixture, and finally the remaining solution of durol diamine is subjected to a phosgenation, optionally after dilution with the same solvent.

7. Method according to claim 1, **characterised in that** for the phosgenation the solution of the durol diamine obtained by the hydrogenation is, after separating the water and the catalyst and optionally after diluting with the same solvent, continuously and intensively mixed in a mechanically operated mixing device with a solution of phosgene in an organic solvent, the resultant reaction mixture is added to a downstream reactor, and there the reaction to form durol diisocyanate is brought to completion by raising the temperature and optionally by the further addition of phosgene.

## Revendications

1. Procédé pour la préparation du durènediisocyanate par réaction du durène avec l'acide nitrique dans l'acide sulfurique en l'absence de solvants organiques, donnant le dinitrodurène qu'on soumet à hydrogénation catalytique, laquelle donne la durènediamine qu'on soumet à phosgénation, ce procédé se caractérisant en ce que a) après la réaction du durène avec l'acide nitrique dans l'acide sulfurique dans un intervalle de température de 10 à 15°C, on dilue par mélange avec de l'eau la phase liquide du mélange de réaction consistant essentiellement en acide sulfurique, b) on soumet la suspension ainsi obtenue à mélange intensif avec un solvant organique aprotonique non miscible à l'eau et inerte à l'égard de l'hydrogène, ce qui provoque la dissolution du dinitrodurène contenu dans le solvant avec formation d'un mélange à deux phases liquides, c) à partir de ce mélange, on sépare par séparation de phases la solution du dinitrodurène dans le solvant organique et, le cas échéant, on la purifie par extraction à l'eau, d) on soumet la solution de dinitrodurène ainsi obtenue, éventuellement après concentration par distillation du solvant, à hydrogénation avec adjonction d'un catalyseur solide insoluble, e) à partir du mélange de réaction obtenu à l'hydrogénation on sépare l'eau et le catalyseur et f) on soumet la solution de durènediamine ainsi obtenue à phosgénation.

2. Procédé selon revendication 1 **caractérisé en ce que**, à partir du mélange de réaction obtenu à la réaction du durène avec l'acide nitrique et avant le mélange avec l'eau on sépare une partie de la phase liquide par filtration ou centrifugation du dinitrodurène.

3. Procédé selon revendication 3 **caractérisé en ce que** l'acide résiduaire séparé du mélange de réaction à la filtration ou centrifugation du dinitrodurène brut est réutilisé pour une réaction consécutive du durène avec l'acide nitrique.

4. Procédé selon revendication 1 **caractérisé en ce que** le solvant organique aprotonique utilisé est le toluène.

5. Procédé selon revendication 1 **caractérisé en ce que** le solvant organique aprotonique utilisé est le chlorobenzène.

6. Procédé selon revendication 1 **caractérisé en ce que**, après l'hydrogénation catalytique du dinitrodurène en solution, on débarrasse totalement le mélange de réaction de l'eau qu'il contient par distillation, on en sépare ensuite le catalyseur et finalement on soumet la solution de durènediamine ainsi obtenue, éventuellement après dilution par le même solvant, à une phosgénation.

7. Procédé selon revendication 1 **caractérisé en ce que**, pour la phosgénation, on soumet la solution de durènediamine obtenue à l'hydrogénation, après séparation de l'eau et du catalyseur, le cas échéant après dilution par le même solvant, à mélange intensif continu dans un appareil de mélange mécanique avec une solution de phosgène dans un solvant organique, on envoie le mélange de réaction formé dans un réacteur placé à la suite où on achève la conversion en durènediisocyanate en augmentant la température et le cas échéant en ajoutant des compléments de phosgène.
